# EUROPEAN PATENT APPLICATION

(11) **EP 4 706 622 A1**
(43) Date of publication of application: **11.03.2026**
(21) Application number: 24815807.3
(22) Date of filing: 24.05.2024
(51) Int. Cl.: A61H 5/00, G06F 3/01, G02B 27/02, G02B 27/01, A61F 9/02

(54) **APPARATUS AND METHOD FOR PROVIDING STEREOPSIS TRAINING**

(30) Priority: 26.05.2023 KR 20230068466
(71) Applicant: NUNAPS INC., Seoul 05505 (KR); The Asan Foundation, Seoul 05505 (KR); UNIVERSITY OF ULSAN FOUNDATION FOR INDUSTRY COOPERATION, Nam-gu Ulsan 44610 (KR)
(72) Inventor: KANG, Dong Wha, Seoul 05505 (KR); LEE, Byung Joo, Seoul 05505 (KR); RYU, Yong Hoe, Seoul 05505 (KR); PARK, Inseok, Seoul 05505 (KR)
(74) Representative: dompatent
(86) International application number: PCT/KR2024/007113
(87) International publication number: WO 2024/248431

(57) **Abstract**

Disclosed is a stereoscopic vision training providing apparatus including a display module, a memory including a first reference image, a second reference image, a plurality of training images, stereoscopic vision information, an input module that receives a response of a trainee, and a control module that outputs the first reference image to a first area of the display module, and outputs the second reference image to a second area of the display module, sets training coordinates based on a coordinate change signal for the first reference image and the second reference image, outputs the plurality of training images with different stereoscopic effects to the display module based on the training coordinates, and performs a stereoscopic vision training operation of receiving a selection response to a training image with a high stereoscopic effect.

## Description

### [TECHNICAL FIELD]

Embodiments of the present disclosure described herein relate to a stereoscopic vision training providing apparatus and a method thereof, and more particularly, relate to a stereoscopic vision training providing apparatus for patients with strabismus, and a method thereof.

### [BACKGROUND ART]

Nowadays, myopia, astigmatism, or strabismus has a high incidence due to the recent development of media. The strabismus among them refers to a state where two eyes are not aligned properly. When patients with strabismus are not treated early, their vision may never be restored, and thus the patients with strabismus in childhood need to be identified and corrected early.

When the strabismus occurs in a child between ages of 2 and 5, the child's two eyes watch different objects from each other, respectively. As a result, because two completely different images are sent to the child's brain, the child experiences visual confusion, where completely different objects appear to be superimposed. Moreover, as the child experiences double vision where one image appears to be in two different places at the same time, the child ignores an image coming from one side to resolve the visual confusion and the double vision. As a result, the child loses the ability to perceive binocular vision, which enables an object to appear three-dimensionally. Furthermore, the child also abandons the use of the eye with strabismus, resulting in amblyopia.

Accordingly, there is a need to develop technology for providing and controlling stereoscopic vision training to improve a user's (trainee's) visual perception ability including stereoscopic sense.

### [DETAILED DESCRIPTION OF THE INVENTION]

### [TECHINICAL PROBLEM]

Embodiments of the present disclosure provide an apparatus providing stereoscopic vision training and a method thereof.

Problems to be solved by the present disclosure are not limited to the problems mentioned above, and other problems not mentioned will be clearly understood by those skilled in the art from the following description.

### [TECHNICAL SOLUTION]

According to an embodiment, a stereoscopic vision training providing apparatus includes a display module, a memory including a first reference image, a second reference image, a plurality of training images, stereoscopic vision information, an input module that receives a response of a trainee, and a control module that outputs the first reference image to a first area of the display module, and outputs the second reference image to a second area of the display module, sets training coordinates based on a coordinate change signal for the first reference image and the second reference image, outputs the plurality of training images with different stereoscopic effects to the display module based on the training coordinates, and performs a stereoscopic vision training operation of receiving a selection response to a training image with a high stereoscopic effect.

According to an embodiment, a stereoscopic vision training providing method includes outputting a first reference image to a first area of a display module, and outputting a second reference image to a second area of the display module, setting training coordinates based on a coordinate change signal for the first reference image and the second reference image, outputting a plurality of training images with different stereoscopic effects to the display module based on the training coordinates, and performing a stereoscopic vision training operation of receiving a selection response to a training image with a high stereoscopic effect.

### [ADVANTAGEOUS EFFECTS OF THE INVENTION]

In accordance with the stereoscopic vision training providing apparatus of the present disclosure, it is possible to proactively perform a training coordinate setting operation through measuring the strabismus angle, thereby increasing the effect of stereoscopic vision training by accurately reflecting the degree of strabismus angle according to the trainee's status while the trainee is training.

Moreover, in accordance with the stereoscopic vision training providing apparatus of the present disclosure, it is possible to provide not only static stereoscopic vision training but also dynamic stereoscopic vision training, thereby increasing the effect of a trainee's stereoscopic vision training.

### [DESCRIPTION OF THE DRAWINGS]

FIG. 1 is a block diagram of a stereoscopic vision training providing apparatus, according to an embodiment of the present disclosure.
FIG. 2 is a diagram for describing an operation of setting training coordinates through measuring a strabismus angle, according to an embodiment of the present disclosure.
FIG. 3 is a diagram showing the type of each trainee when a strabismus angle is measured, according to an embodiment of the present disclosure.
FIG. 4 is a diagram showing first training of a stereoscopic vision training operation, according to an embodiment of the present disclosure.
FIG. 5 is a diagram showing second training of a stereoscopic vision training operation, according to an embodiment of the present disclosure.
FIG. 6 is a diagram showing third training of a stereoscopic vision training operation, according to an embodiment of the present disclosure.
FIG. 7 is a diagram showing fourth training of a stereoscopic vision training operation, according to an embodiment of the present disclosure.
FIG. 8 is a diagram showing training images, according to an embodiment of the present disclosure.
FIG. 9 is a table showing stereoscopic vision of a training image according to difficulty of first training and fourth training, according to an embodiment of the present disclosure.
FIG. 10 is a table showing stereoscopic vision of a training image according to the difficulty of second training, according to an embodiment of the present disclosure.
FIG. 11 is a table showing stereoscopic vision of a training image according to the difficulty of third training, according to an embodiment of the present disclosure.
FIG. 12 is a diagram showing training coordinates, according to an embodiment of the present disclosure.
FIG. 13 is a diagram showing training coordinates according to difficulty and stereoscopic vision, according to an embodiment of the present disclosure.
FIG. 14 is a flowchart showing a method of providing stereoscopic vision training, according to an embodiment of the present disclosure.
FIG. 15 is a flowchart showing a method for setting training coordinates of the present disclosure.

### [BEST MODE]

The same reference numerals denote the same elements throughout the present disclosure. The present disclosure does not describe all elements of embodiments. Well-known content or redundant content in which embodiments are the same as one another will be omitted in a technical field to which the present disclosure belongs. A term such as 'unit, module, member, or block' used in the specification may be implemented with software or hardware. According to embodiments, a plurality of 'units, modules, members, or blocks' may be implemented with one component, or a single 'unit, module, member, or block' may include a plurality of components.

Throughout this specification, when it is supposed that a portion is "connected" to another portion, this includes not only a direct connection, but also an indirect connection. The indirect connection includes being connected through a wireless communication network.

Furthermore, when a portion "comprises" a component, it will be understood that it may further include another component, without excluding other components unless specifically stated otherwise.

Throughout this specification, when it is supposed that a member is located on another member "on", this includes not only the case where one member is in contact with another member but also the case where another member is present between two other members.

Terms such as 'first', 'second', and the like are used to distinguish one component from another component, and thus the component is not limited by the terms described above.

Unless there are obvious exceptions in the context, a singular form includes a plural form.

In each step, an identification code is used for convenience of description. The identification code does not describe the order of each step. Unless the context clearly states a specific order, each step may be performed differently from the specified order.

Hereinafter, operating principles and embodiments of the present disclosure will be described with reference to the accompanying drawings.

In this specification, an 'apparatus according to an embodiment of the present disclosure' includes all various devices capable of providing results to a user by performing arithmetic processing. For example, the apparatus according to an embodiment of the present disclosure may include all of a computer, a server device, and a portable terminal, or may be in any one form.

Here, for example, the computer may include a notebook computer, a desktop computer, a laptop computer, a tablet PC, a slate PC, and the like, which are equipped with a web browser.

The server device may be a server that processes information by communicating with an external device and may include an application server, a computing server, a database server, a file server, a game server, a mail server, a proxy server, and a web server.

For example, the portable terminal may be a wireless communication device that guarantees portability and mobility, and may include all kinds of handheld-based wireless communication devices such as a smartphone, a personal communication system (PCS), a global system for mobile communication (GSM), a personal digital cellular (PDC), a personal handyphone system (PHS), a personal digital assistant (PDA), International Mobile Telecommunication (IMT)-2000, a code division multiple access (CDMA)-2000, W-Code Division Multiple Access (W-CDMA), and Wireless Broadband Internet terminal (Wibro) terminal, and a wearable device such as a timepiece, a ring, a bracelet, an anklet, a necklace, glasses, a contact lens, or a head-mounted device (HMD).

Functions related to artificial intelligence according to an embodiment of the present disclosure are operated through a processor and a memory. The processor may consist of one or more processors. In this case, the one or more processors may be a general-purpose processor (e.g., a CPU, an AP, or a digital signal processor (DSP)), a graphics-dedicated processor (e.g., a GPU or a vision processing unit (VPU)), or an artificial intelligence-dedicated processor (e.g., an NPU). Under control of the one or more processors, input data may be processed depending on an artificial intelligence model, or a predefined operating rule stored in the memory. Alternatively, when the one or more processors are artificial intelligence-dedicated processors, the artificial intelligence-dedicated processor may be designed with a hardware structure specialized for processing a specific artificial intelligence model.

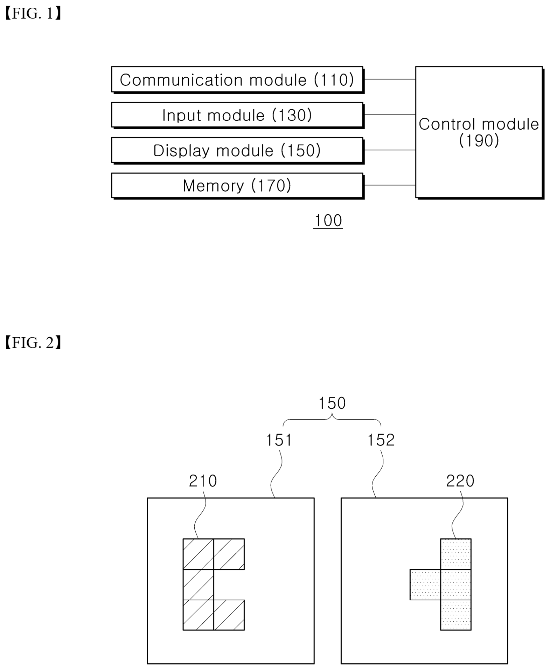

FIG. 1 is a block diagram of a stereoscopic vision training providing apparatus, according to an embodiment of the present disclosure. Referring to FIG. 1, a stereoscopic vision training providing apparatus 100 may include a communication module 110, an input module 130, a display module 150, a memory 170, and a control module 190. The components shown in FIG. 1 are not essential in implementing the stereoscopic vision training providing apparatus 100. The stereoscopic vision training providing apparatus 100 described herein may have more or fewer components than those listed above.

The stereoscopic vision training providing apparatus 100 according to an embodiment of the present disclosure may include various devices capable of performing calculation processing. For example, the stereoscopic vision training providing apparatus 100 may include a desktop PC, a mobile phone, a smart phone, a laptop computer, personal digital assistants (PDA), a portable multimedia player (PMP), a slate PC, a tablet PC, an ultra-book, a wearable device, and the like.

In the meantime, the stereoscopic vision training providing apparatus 100 may include a display device of a mobile phone capable of being mounted and used on a head-mounted device such as a head mounted display (HMD) mounted on a trainee's head to display an image, smart glasses, or smart goggles.

The communication module 110 performs wired or wireless communication with at least one external device (a server, etc.). In particular, when wireless communication is performed, wireless signals are exchanged over a communication network based on wireless Internet technologies.

For example, the wireless Internet technologies includes wireless LAN (WLAN), Wireless-Fidelity (Wi-Fi), Wi-Fi Direct, digital living network alliance (DLNA), wireless broadband (WiBro), world interoperability for microwave access (WiMAX), high speed downlink packet access (HSDPA), high speed uplink packet access (HSUPA), long term evolution (LTE), long term evolution-advanced (LTE-A), and the like. The stereoscopic vision training providing apparatus 100 transmits and receives data depending on at least one wireless Internet technology within a range including Internet technologies not listed above.

The communication module 110 may be used for short range communication, and may support short-range communication by using at least one of Bluetooth^{™}, radio frequency identification (RFID), infrared data association (IrDA), ultra wideband (UWB), ZigBee, near field communication (NFC), Wireless-Fidelity (Wi-Fi), Wi-Fi Direct, and wireless universal serial bus (Wireless USB) technologies. In this case, the short range wireless communication network may be wireless personal area networks.

The input module 130 may obtain a signal corresponding to a trainee's input. For example, the input module 130 may obtain the trainee's input for measuring a strabismus angle or performing stereoscopic vision training, a response to an operation of measuring the strabismus angle and an operation of stereoscopic vision training, which are provided through the display module 150, or the like.

In this case, the input module 130 may include a keyboard, a keypad, a button, a jog shuttle, a wheel, and the like. Besides, the trainee's input in the input module 130 may be, for example, pressing a button, touching, and dragging.

The input module 130 may be configured as a separate module connected to the stereoscopic vision training providing apparatus 100 in a wired or wireless manner. For example, the stereoscopic vision training providing apparatus 100 may provide a trainee with images for measuring a strabismus angle or performing stereoscopic vision training through the display module 150 mounted on the trainee's head, and may receive a response from the trainee through the input module 130 implemented as a separate module given to the trainee's hand.

The display module 150 outputs videos or images. For example, the display module 150 may include an LCD, an OLED display, an AMOLED display, and the like. In an embodiment, when the display module 150 is implemented as a touch screen, the display module 150 may serve as the input module 130. In this case, the separate input module 130 may not be provided depending on a selection, or the input module 130 performing limited functions such as a volume control button, a power button, and a home button may be provided. Moreover, the display module 150 may be provided in a form of a video output port that transmits image information to an external display device.

The display module 150 may include a first display and a second display, which respectively correspond to the trainee's eyes (a left eye and a right eye). Here, the first display may output a first image, and the second display may output a second image.

In the meantime, the present disclosure is not limited thereto, and the display module 150 may be implemented as a single display. In this case, the display module 150 may output the first image in a left area of the display and may output the second image in a right area of the display.

The memory 170 stores various pieces of data (information) as well as at least a piece of data (information) and at least one process, which are necessary to provide a stereoscopic vision training method. For example, a program for performing training, at least a piece of trainee information (personal information, visual information, response information, training results, or the like), various reference values that is a basis for measuring the strabismus angle, measurement images, a training image, or the like may be stored as data. Besides, the memory 170 may store various instructions, algorithms, or the like for performing a stereoscopic vision training operation.

Moreover, the memory 170 may include a storage medium of at least one type of a flash memory type, a hard disk type, a multimedia card micro type, a card type memory (e.g., SD memory, XD memory, etc.), a random access memory (RAM), a static random access memory (SRAM), a read-only memory (ROM), an electrically erasable programmable read-only memory (EEPROM), a programmable read-only memory (PROM), a magnetic memory, a magnetic disk, an optical disc, etc. Besides, the memory 170 may store information temporarily, permanently, or semi-permanently, and may be provided in an embedded type or a removable type.

The control module 190 may control configurations within the stereoscopic vision training providing apparatus 100 or may process and calculate various pieces of information. Under control of the control module 190, the stereoscopic vision training operation may be performed based on at least one process stored in the memory 170.

Before performing the stereoscopic vision training operation, the control module 190 may perform a training coordinate setting operation by measuring the trainee's strabismus angle. In detail, the control module 190 may output a first reference image to an area corresponding to a left eye through the display module 150 and may output a second reference image to an area corresponding to a right eye through the display module 150. In this case, when a trainee with a normal eye looks at the first reference image and the second reference image together, the first reference image and the second reference image may be output such that a specific shape (e.g., a square, a heart, or the like) is displayed. In the meantime, when a trainee with a strabismus eye looks at the first reference image and the second reference image together, there may be a horizontal separation distance, and thus they may not be seen as the specific shape. In this case, the trainee may input a coordinate change signal for completing a specific shape by moving the first reference image or the second reference image in the left and right directions through the input module 130. The control module 190 may calculate whether the trainee's strabismus is left or right, and the extent of the strabismus angle based on the input coordinate change signal. In addition, the control module 190 may set training coordinates based on the calculated strabismus angle. For example, the control module 190 may set the training coordinates such that an image for stereoscopic vision training is output to the display module 150 so as to correspond to the viewing angle of each of a normal eye and/or a strabismus eye of the trainee by using the calculated strabismus angle.

Moreover, the control module 190 may perform a stereoscopic vision training operation based on the set training coordinates. For example, the control module 190 may perform a static stereoscopic vision training operation and a dynamic stereoscopic vision training operation. The static stereoscopic vision training operation refers to a training process in which a trainee sequentially selects an image, which appears to protrude further forward, from among a plurality of stationary training images. Moreover, the dynamic stereoscopic vision training operation refers to a training process in which the trainee selects an image, which appears to protrude further forward, from among a plurality of moving training images.

The control module 190 may be implemented with software, hardware, or a combination thereof. For example, in a hardware manner, the control module 190 may be implemented with a field programmable gate array (FPGA) or an application specific integrated circuit (ASIC), a semiconductor chip, or other various types of electronic circuits. Also, for example, in a software manner, the control module 190 may be implemented with various computer languages or a logic program executed depending on the above-described hardware.

Unless otherwise stated in the following description, it may be understood that an operation of the stereoscopic vision training providing apparatus 100 is performed under the control of the control module 190.

As such, the stereoscopic vision training providing apparatus 100 according to an embodiment of the present disclosure may proactively perform a training coordinate setting operation through measuring the strabismus angle, thereby increasing the effect of stereoscopic vision training by accurately reflecting the degree of strabismus angle according to the trainee's status while the trainee is training.

Moreover, the stereoscopic vision training providing apparatus 100 according to an embodiment of the present disclosure may provide not only static stereoscopic vision training but also dynamic stereoscopic vision training, thereby increasing the effect of a trainee's stereoscopic vision training.

In the meantime, according to an embodiment, the stereoscopic vision training providing apparatus 100 may be implemented to perform a training coordinate setting operation and a stereoscopic vision training operation through an external device. For example, when the stereoscopic vision training providing apparatus 100 is implemented as a server, the stereoscopic vision training providing apparatus 100 may perform the training coordinate setting operation and the stereoscopic vision training operation by communicating with an external device through the communication module 110. In this case, an operation of using the input module 130 and the display module 150 of the stereoscopic vision training providing apparatus 100 described in this specification may be replaced with an operation of using a display module and an input module of the external device through the communication module 110.

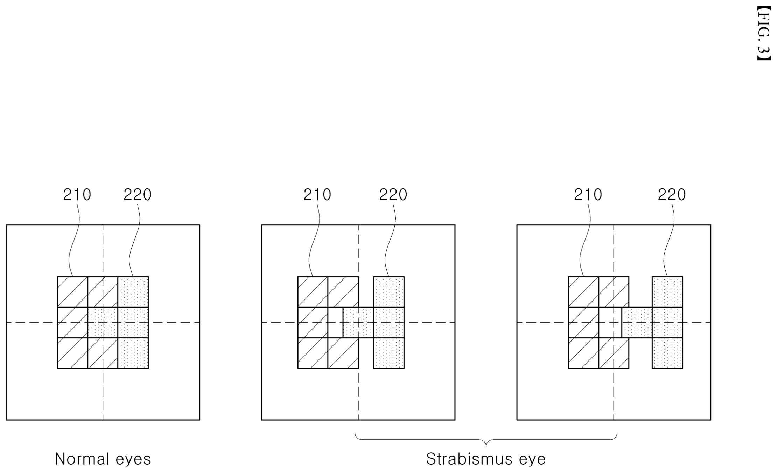

FIG. 2 is a diagram for describing an operation of setting training coordinates through measuring a strabismus angle, according to an embodiment of the present disclosure. FIG. 3 is a diagram showing the type of each trainee when a strabismus angle is measured, according to an embodiment of the present disclosure.

Referring to FIG. 2, the display module 150 may include a first display 151 and a second display 152. In this disclosure, the first display 151 and the second display 152 may be implemented as a plurality of physically separate displays or may be implemented as a concept of an area divided into a left area and a right area within one display.

In an embodiment, the first display 151 may output a first reference image 210 corresponding to a left eye, and the second display 152 may output a second reference image 220 corresponding to a right eye. The first reference image 210 and the second reference image 220 may be different images from each other. For example, referring to FIG. 2, the first reference image 210 is an image having a shape of ' 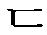 ', and the second reference image 220 is an image having a shape of ' 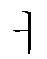 '.

Moreover, when a trainee with normal eyes looks at the first reference image 210 and the second reference image 220, the first reference image 210 and the second reference image 220 may be output such that the first reference image 210 and the second reference image 220 appear together to form a specific shape. For example, referring to FIG. 3, when viewed by a trainee with normal eyes, the first reference image 210 and the second reference image 220 may be output to appear as a square obtained by combining ' 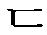 ' and ' 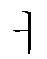 '. Hereinafter, this specific shape is referred to as a "reference shape".

In the meantime, when a trainee with a strabismus eye looks at the first reference image 210 and the second reference image 220, the trainee may perceive the first reference image 210 and the second reference image 220 to be different from the above-described shape. For example, referring to FIG. 3, a trainee with a strabismus eye may perceive that the first reference image 210 or the second reference image 220 is more spaced in from the left or right, and may not perceive the first reference image 210 or the second reference image 220 as a square obtained by combing ' 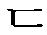 ' and ' 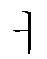 '.

Accordingly, the stereoscopic vision training providing apparatus 100 may request the trainee to move the first reference image 210 or the second reference image 220 to the left and right through the display module 150 such that the first reference image 210 and the second reference image 220 have a reference shape. Furthermore, the stereoscopic vision training providing apparatus 100 may receive a coordinate change signal for moving the first reference image 210 or the second reference image 220 in the left and right directions from the trainee through the input module 130. Also, when a signal indicating that the coordinate change has been completed is additionally received from the trainee, training coordinates may be set based on the location of the first reference image 210 or the second reference image 220.

In detail, the stereoscopic vision training providing apparatus 100 may calculate a boundary area including the first reference image 210 and the second reference image 220 and may calculate the center coordinates of the boundary area. In this case, the boundary area includes the first reference image 210 and the second reference image 220 and is a shape such as a square or circle with a minimum size. In this case, the origin, which is the basis, is the center coordinates of the boundary area of the first reference image 210 and the second reference image 220 that do not move in the left or right direction. In other words, the origin is the center coordinates of the boundary area when a trainee with normal eyes looks at the first reference image 210 and the second reference image 220. The stereoscopic vision training providing apparatus 100 may set training coordinates based on the calculated center coordinates. Moreover, the stereoscopic vision training providing apparatus 100 may perform a stereoscopic vision training operation based on the set training coordinates.

In an embodiment, for high accuracy, the stereoscopic vision training providing apparatus 100 may perform the above-described operations multiple times. In other words, the stereoscopic vision training providing apparatus 100 may output the first reference image 210 and the second reference image 220 to the trainee, may receive a coordinate change signal to perform an operation of calculating the center coordinates of the boundary area multiple times (e.g., three times), and may set training coordinates using the average value of the calculated plurality of center coordinates.

In the meantime, shapes of the first reference image 210 and the second reference image 220 are not limited to the examples shown in FIGS. 2 and 3 and may be implemented as various images. Moreover, in FIGS. 2 and 3, the first reference image 210 and the second reference image 220 are shown and described as being different images, but the present disclosure is not limited thereto and may be implemented as the same image.

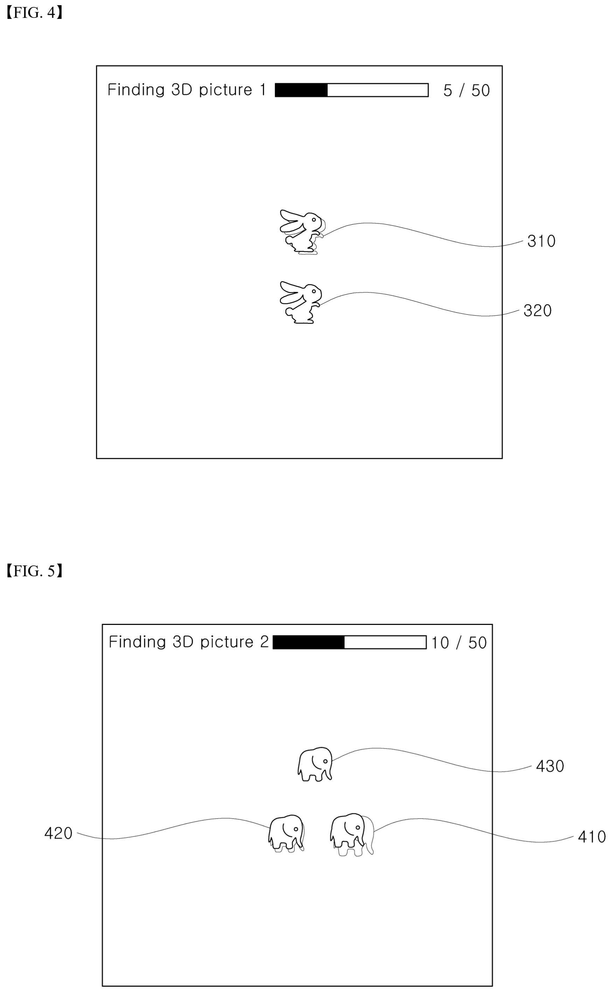

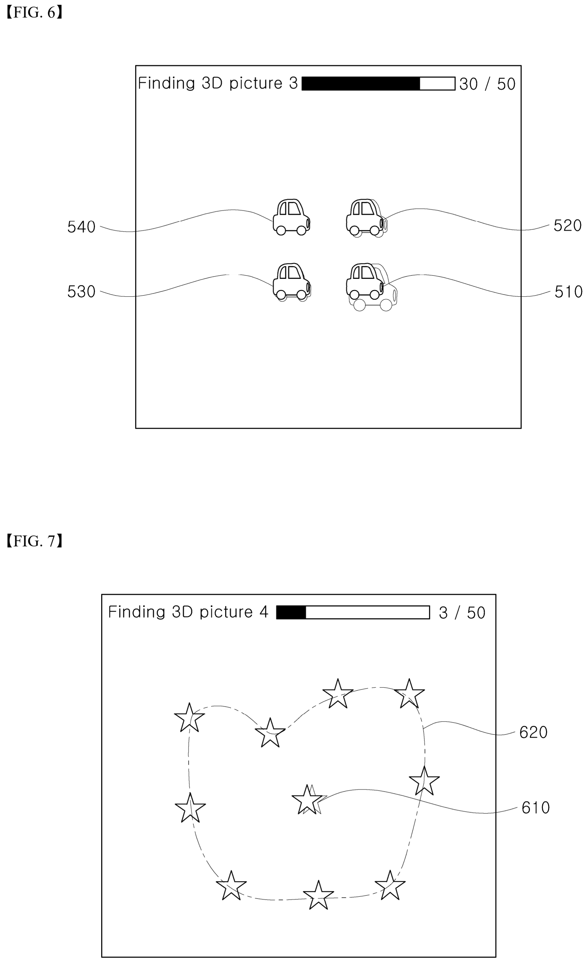

Hereinafter, the static stereoscopic vision training operation will be described based on FIGS. 4 to 6, and the dynamic stereoscopic vision training operation will be described based on FIG. 7.

FIG. 4 is a diagram showing first training of a stereoscopic vision training operation, according to an embodiment of the present disclosure. Referring to FIG. 4, first training of a stereoscopic vision training operation is performed by using two stationary training images 310 and 320. Moreover, FIG. 4 shows an image perceived when a trainee looks at the display module 150 with both eyes during the first training.

In an embodiment, the stereoscopic vision training providing apparatus 100 may output the first training image 310 and the second training image 320 on both the first display 151 and the second display 152 through the display module 150. In this case, pieces of stereoscopic information of the first training image 310 and the second training image 320 may be different from each other. For example, because the first training image 310 has a stereoscopic vision value higher than the second training image 320, the first training image 310 is an image requiring that the first training image 310 appears to protrude further forward when the trainee looks at both the first display 151 and the second display 152.

To reflect the stereoscopic effect of each of the first training image 310 and the second training image 320, the stereoscopic vision training providing apparatus 100 may adjust at least one of the training coordinates of the first training image 310 and the second training image 320 based on stereoscopic vision information. For example, the stereoscopic vision training providing apparatus 100 may adjust the training coordinates of the first training image 310 such that the first training image 310 appears to protrude further forward. According to an embodiment, when an image (e.g., the second training image 320) having the stereoscopic vision value of 0 is present, the training coordinates of the corresponding image may not be adjusted.

The stereoscopic vision training providing apparatus 100 may output the first training image 310 and the second training image 320 on the display module 150 based on the adjusted training coordinates. Besides, the stereoscopic vision training providing apparatus 100 may receive an input for selecting the training images 310 and 320 from the trainee through the input module 130 in descending order of stereoscopic effect. The stereoscopic vision training providing apparatus 100 may calculate the trainee's training score in consideration of whether the trainee's input is correct, the time required to enter an input, or the like.

FIG. 5 is a diagram showing second training of a stereoscopic vision training operation, according to an embodiment of the present disclosure. Referring to FIG. 5, second training of a stereoscopic vision training operation is performed by using three stationary training images 410, 420, and 430. Moreover, FIG. 5 shows an image perceived when a trainee looks at the display module 150 with both eyes during the second training.

In an embodiment, the stereoscopic vision training providing apparatus 100 may output the first training image 410, the second training image 420, and the third training image 430 on both the first display 151 and the second display 152 through the display module 150. In this case, pieces of stereoscopic information of the first training image 410, the second training image 420, and the third training image 430 may be different from each other. For example, because the first training image 410 has a stereoscopic vision value higher than the second training image 420 and the third training image 430, the first training image 410 is an image requiring that the first training image 410 appears to protrude further forward when the trainee looks at both the first display 151 and the second display 152. Furthermore, because the second training image 420 has a stereoscopic vision value higher than the third training image 430, the second training image 420 is an image requiring that the second training image 420 appears to protrude further forward when the trainee looks at both the first display 151 and the second display 152.

To reflect the stereoscopic effect of each of the first training image 410, the second training image 420, and the third training image 430, the stereoscopic vision training providing apparatus 100 may adjust at least one of the training coordinates of the first training image 410, the second training image 420, and the third training image 430 based on stereoscopic vision information. For example, the stereoscopic vision training providing apparatus 100 may adjust the training coordinates of the first training image 410 and the second training image 420 such that the first training image 410 appears to protrude the most forward, and the second training image 420 appears to protrude the second most forward. According to an embodiment, when an image (e.g., the third training image 430) having the stereoscopic vision value of 0 is present, the training coordinates of the corresponding image may not be adjusted.

The stereoscopic vision training providing apparatus 100 may output the first training image 410, the second training image 420, and the third training image 430 on the display module 150 based on the adjusted training coordinates. Besides, the stereoscopic vision training providing apparatus 100 may receive an input for selecting the training images 410, 420, and 430 from the trainee through the input module 130 in descending order of stereoscopic effect. The stereoscopic vision training providing apparatus 100 may calculate the trainee's training score in consideration of whether the trainee's input is correct, the time required to enter an input, or the like.

FIG. 6 is a diagram showing third training of a stereoscopic vision training operation, according to an embodiment of the present disclosure. Referring to FIG. 6, third training of a stereoscopic vision training operation is performed by using four stationary training images 510, 520, 530, and 540. Moreover, FIG. 6 shows an image perceived when a trainee looks at the display module 150 with both eyes during the third training.

In an embodiment, the stereoscopic vision training providing apparatus 100 may output the first training image 510, the second training image 520, the third training image 530, and the fourth training image 540 on both the first display 151 and the second display 152 through the display module 150. In this case, pieces of stereoscopic information of the first training image 510, the second training image 520, the third training image 530, and the fourth training image 540 may be different from each other. For example, stereoscopic vision may be high in the order of 'the first training image 510-the second training image 520-the third training image 530-the fourth training image 540'. Accordingly, it may be required to appear to protrude forward in the order of 'the first training image 510-the second training image 520-the third training image 530-the fourth training image 540'.

To reflect the stereoscopic effect of each of the first training image 510, the second training image 520, the third training image 530, and the fourth training image 540, the stereoscopic vision training providing apparatus 100 may adjust at least one of the training coordinates of the first training image 510, the second training image 520, the third training image 530, and the fourth training image 540 based on stereoscopic vision information. For example, the stereoscopic vision training providing apparatus 100 may adjust the training coordinates of the first training image 510, the second training image 520, and the third training image 530 such that they appear to protrude forward in the order of 'the first training image 510-the second training image 520-the third training image 530-the fourth training image 540'. According to an embodiment, when an image (e.g., the fourth training image 540) having the stereoscopic vision value of 0 is present, the training coordinates of the corresponding image may not be adjusted.

The stereoscopic vision training providing apparatus 100 may output the first training image 510, the second training image 520, the third training image 530, and the fourth training image 540 on the display module 150 based on the adjusted training coordinates. Besides, the stereoscopic vision training providing apparatus 100 may receive an input for selecting the training images 510, 520, 530, and 540 from the trainee through the input module 130 in descending order of stereoscopic effect. The stereoscopic vision training providing apparatus 100 may calculate the trainee's training score in consideration of whether the trainee's input is correct, the time required to enter an input, or the like.

As such, the stereoscopic vision training providing apparatus 100 according to an embodiment of the present disclosure provides a static stereoscopic vision training operation based on training coordinates according to strabismus angle evaluation, thereby improving static stereoscopic vision ability even when the trainee's eyes are currently uncorrected.

In the meantime, FIGS. 4 to 6 illustrate and describe that the stereoscopic vision training providing apparatus 100 performs static stereoscopic vision training by using two to four training images, but the present disclosure is not limited thereto. For example, it is obvious that the stereoscopic vision training providing apparatus 100 may be implemented to perform the static stereoscopic vision training by using five or more training images.

In addition, FIGS. 4 to 6 illustrate and describe that the stereoscopic vision training providing apparatus 100 provides the static stereoscopic vision training operation of performing all of the first training, the second training, and the third training, but the present disclosure is not limited thereto. For example, the stereoscopic vision training providing apparatus 100 may be implemented to provide a static stereoscopic vision training operation of performing at least one of the first training, the second training, and the third training.

FIG. 7 is a diagram showing fourth training of a stereoscopic vision training operation, according to an embodiment of the present disclosure. Referring to FIG. 7, fourth training of a stereoscopic vision training operation is performed by using ten moving training images 610 and 620. Moreover, FIG. 7 shows an image perceived when a trainee looks at the display module 150 with both eyes during the fourth training.

In an embodiment, the stereoscopic vision training providing apparatus 100 may output the first training image 610 and the plurality of second training images 620 on both the first display 151 and the second display 152 through the display module 150. In this case, stereoscopic information of the first training image 610 may be different from stereoscopic information of each of the second training image 620. For example, because the first training image 610 has a stereoscopic vision value higher than the plurality of second training images 620, the first training image 610 is an image requiring that the first training image 610 appears to protrude further forward when the trainee looks at both the first display 151 and the second display 152.

To reflect the stereoscopic effect of the first training image 310 and each of the plurality of second training images 620, the stereoscopic vision training providing apparatus 100 may adjust at least one of the training coordinates of the first training image 310 and the plurality of second training images 620 based on stereoscopic vision information. For example, the stereoscopic vision training providing apparatus 100 may adjust the training coordinates of the first training image 610 such that the first training image 610 appears to protrude further forward. According to an embodiment, when an image (e.g., the plurality of second training images 620) having the stereoscopic vision value of 0 is present, the training coordinates of the corresponding image may not be adjusted.

The stereoscopic vision training providing apparatus 100 may output the first training image 610 and the plurality of second training images 620 on the display module 150 based on the adjusted training coordinates. Moreover, the stereoscopic vision training providing apparatus 100 may receive an input for selecting an image with a high stereoscopic effect among moving images from the trainee through the input module 130. The stereoscopic vision training providing apparatus 100 may calculate the trainee's training score in consideration of whether the trainee's input is correct, the time required to enter an input, or the like.

As such, the stereoscopic vision training providing apparatus 100 according to an embodiment of the present disclosure provides a dynamic stereoscopic vision training operation based on training coordinates according to strabismus angle evaluation, thereby improving dynamic stereoscopic vision ability even when the trainee's eyes are currently uncorrected.

The stereoscopic vision training providing apparatus 100 of the present disclosure may perform each of the first training, the second training, the third training, and the fourth training of the stereoscopic vision training operation multiple times. For example, the stereoscopic vision training providing apparatus 100 may perform each of the first training, the second training, the third training, and the fourth training 50 times. In addition, the stereoscopic vision training providing apparatus 100 may adjust the difficulty of the next training based on training scores of the first training, the second training, the third training, and the fourth training, which are performed first. For example, the stereoscopic vision training providing apparatus 100 may adjust difficulty by changing the difference in stereoscopic vision between training images, or changing training images into images that are easier or more difficult to identify the stereoscopic effect.

In the meantime, FIGS. 3 to 7 illustrate that the stereoscopic vision training providing apparatus 100 receives an input for selecting a training image in descending order of a stereoscopic effect in a stereoscopic vision training operation, but the present disclosure is not limited thereto. For example, the stereoscopic vision training providing apparatus 100 may be implemented such that training images are selected in ascending order of stereoscopic effect, or may be implemented such that an image having the highest or lowest stereoscopic effect is alternately selected whenever an image is selected.

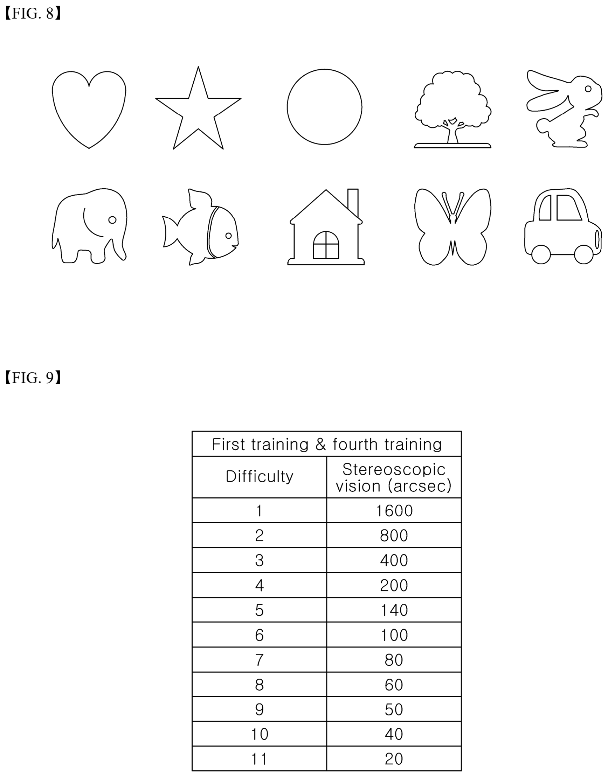

FIG. 8 is a diagram showing training images, according to an embodiment of the present disclosure. Referring to FIG. 8, training images may be implemented in various shapes such as a heart shape, a star shape, a circle shape, a tree shape, a rabbit shape, an elephant shape, a fish shape, a house shape, a butterfly shape, a car shape, or the like.

In an embodiment, each of the training images may have a predetermined size. The predetermined size may be set based on a display size of the display module 150, a distance between a trainee and the display, or the like.

In an embodiment, the stereoscopic vision training providing apparatus 100 may be implemented to use only the same training image within a piece of training. For example, referring to FIG. 4, in the first training of the static stereoscopic vision training operation, the stereoscopic vision training providing apparatus 100 may use training images of a rabbit shape. Moreover, referring to FIG. 5, in the second training of the static stereoscopic vision training operation, the stereoscopic vision training providing apparatus 100 may use training images of an elephant shape. Furthermore, referring to FIG. 6, in the third training of the static stereoscopic vision training operation, the stereoscopic vision training providing apparatus 100 may use training images of a car shape. Besides, referring to FIG. 7, in the fourth training of the dynamic stereoscopic vision training operation, the stereoscopic vision training providing apparatus 100 may use training images of a star shape. In the meantime, the example is only an embodiment, and a training image used for each training type may be set in various ways.

In another embodiment, when performing training multiple times, the stereoscopic vision training providing apparatus 100 may use different training images for each session. For example, the stereoscopic vision training providing apparatus 100 may use training images of a rabbit shape in a first session of the first training and may use training images of a star shape in the next session.

In an embodiment, the stereoscopic vision training providing apparatus 100 may set difficulty based on the symmetry of the training image. In detail, a training image (e.g., a circle shape) with vertical and horizontal symmetry is relatively easy to feel the stereoscopic effect compared with an image with only vertical symmetry, an image (e.g., a star shape, a heart shape, a tree shape, or a butterfly shape) with only horizontal symmetry, or a training image (a rabbit shape, an elephant shape, a fish shape, a house shape, or a car shape) without symmetry. In this respect, the stereoscopic vision training providing apparatus 100 may be set such that images have gradually increasing difficulty in the order of 'a training image with vertical and horizontal symmetry - an image with vertical symmetry or an image with horizontal symmetry - an image without symmetry'. Furthermore, the stereoscopic vision training providing apparatus 100 may control the difficulty in a stereoscopic vision training operation in consideration of the difficulty of a training image.

FIG. 9 is a table showing stereoscopic vision of a training image according to difficulty of first training and fourth training, according to an embodiment of the present disclosure. The first training and the fourth training use training images with two types of stereoscopic effects, and thus the table indicates stereoscopic vision for each difficulty of an image (e.g., the first training image 310 in FIG. 4 or the first training image 610 in FIG. 7) with high stereoscopic effect.

Referring to FIG. 9, in the first training and the fourth training from the first difficulty to the eleventh difficulty, the stereoscopic vision of each of the first training images 310 and 610 may be identified. For example, in the first training and the fourth training of the first difficulty, the stereoscopic vision of each of the first training images 310 and 610 is 1600 arcsec. Moreover, in the first training and the fourth training of the second difficulty, the stereoscopic vision of each of the first training images 310 and 610 is 800 arcsec. Furthermore, although not shown in FIG. 9, the stereoscopic vision of each of the second training images 320 and 620 may be set to a fixed value (e.g., 0 arcsec).

As such, as the difficulty increases, a difference in stereoscopic vision between the first training images 310 and 610 and the second training images 320 and 620 is reduced.

In the meantime, in the table of FIG. 9, the number of difficulties and information about pieces of stereoscopic vision according to difficulty are not limited to the example. For example, it is obvious that the number of difficulties may be smaller or greater than 11 and stereoscopic vision values according to difficulty may be set to values different from values in the examples.

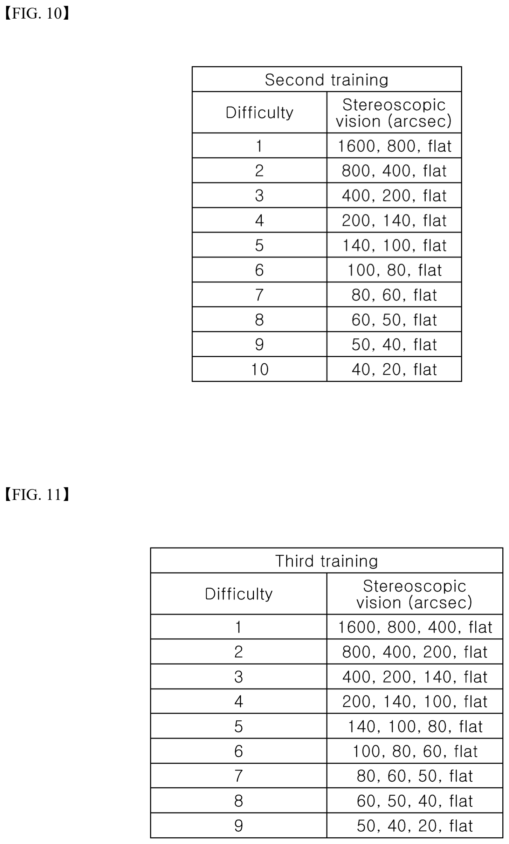

FIG. 10 is a table showing stereoscopic vision of a training image according to the difficulty of the second training, according to an embodiment of the present disclosure. The second training uses training images with three types of stereoscopic effects, and thus the table indicates stereoscopic vision for each difficulty of each of the first training image 410, the second training image 420, and the third training image 430.

Referring to FIG. 10, in the second training from the first difficulty to the tenth difficulty, the stereoscopic vision of each of the first training image 410, the second training image 420, and the third training image 430 may be identified sequentially. For example, in the second training of the first difficulty, the stereoscopic vision of the first training image 410 is 1600 arcsec; the stereoscopic vision of the second training image 420 is 800 arcsec; and, the stereoscopic vision of the third training image 430 is 0 arcsec. For example, in the second training of the second difficulty, the stereoscopic vision of the first training image 410 is 800 arcsec; the stereoscopic vision of the second training image 420 is 400 arcsec; and, the stereoscopic vision of the third training image 430 is 0 arcsec.

As such, as the difficulty increases, the difference in stereoscopic vision between the first training image 410, the second training image 420, and the third training image 430 decreases, and the average value of stereoscopic vision values also decreases.

In the meantime, in the table of FIG. 10, the number of difficulties and information about pieces of stereoscopic vision according to difficulty are not limited to the example. For example, it is obvious that the number of difficulties may be smaller or greater than 10 and stereoscopic vision values according to difficulty may be set to values different from the examples.

FIG. 11 is a table showing stereoscopic vision of a training image according to the difficulty of the third training, according to an embodiment of the present disclosure. The third training uses training images with four types of stereoscopic effects, and thus the table indicates stereoscopic vision for each difficulty of each of the first training image 510, the second training image 520, the third training image 530, and the fourth training image 540.

Referring to FIG. 11, in the third training from the first difficulty to the ninth difficulty, the stereoscopic vision of each of the first training image 510, the second training image 520, the third training image 530, and the fourth training image 540 may be identified sequentially. For example, in the third training of the first difficulty, the stereoscopic vision of the first training image 510 is 1600 arcsec; the stereoscopic vision of the second training image 520 is 800 arcsec; the stereoscopic vision of the third training image 530 is 400 arcsec; and, the stereoscopic vision of the fourth training image 540 is 0 arcsec. Moreover, in the third training of the second difficulty, the stereoscopic vision of the first training image 510 is 800 arcsec; the stereoscopic vision of the second training image 520 is 400 arcsec; the stereoscopic vision of the third training image 530 is 200 arcsec; and, the stereoscopic vision of the fourth training image 540 is 0 arcsec.

As such, as the difficulty increases, the difference in stereoscopic vision between the first training image 510, the second training image 520, the third training image 530, and the fourth training image 540 decreases, and the average value of stereoscopic vision values also decreases.

In the meantime, in the table of FIG. 11, the number of difficulties and information about pieces of stereoscopic vision according to difficulty are not limited to the example. For example, it is obvious that the number of difficulties may be smaller or greater than 9 and stereoscopic vision values according to difficulty may be set to values different from values in the examples.

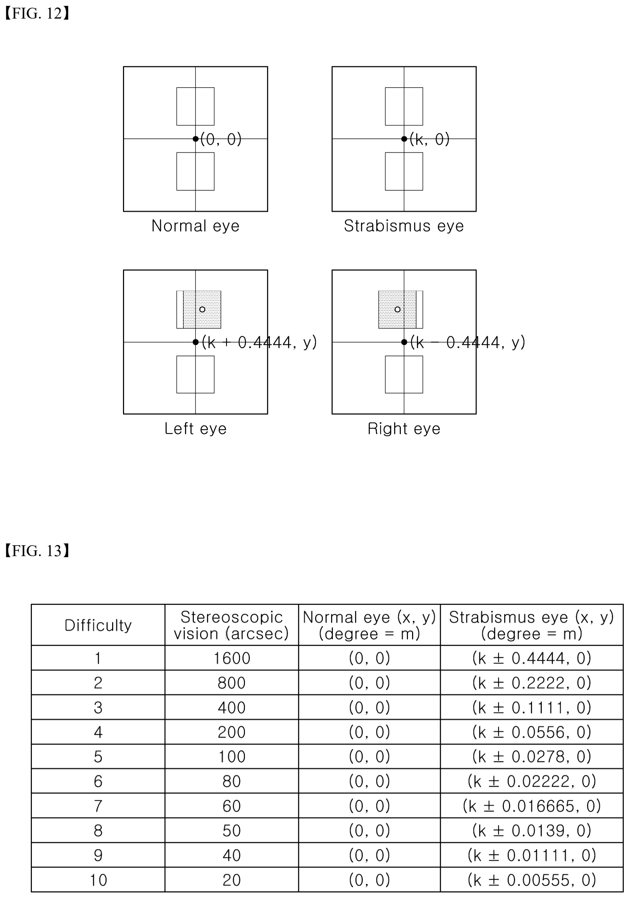

FIG. 12 is a diagram showing training coordinates, according to an embodiment of the present disclosure. FIG. 13 is a diagram showing training coordinates according to difficulty and stereoscopic vision, according to an embodiment of the present disclosure.

Referring to FIG. 12, in an operation of setting training coordinates through measuring a strabismus angle, the training coordinates of a trainee with normal eyes may be set to (0,0). In the meantime, as described in FIG. 2, training coordinates of a trainee with a strabismus eye may be set based on a coordinate change signal for moving the first reference image 210 or the second reference image 220 in left and right directions. For example, when a trainee enters a coordinate change signal for moving the first reference image 210 or the second reference image 220 by 'k', training coordinates may be set to (k,0).

In an embodiment, before performing training, the stereoscopic vision training providing apparatus 100 may adjust training coordinates based on stereoscopic vision information of a training image used for the corresponding training. In this case, the stereoscopic vision training providing apparatus 100 may adjust training coordinates of an area corresponding to a strabismus eye among a normal eye and the strabismus eye. For example, when the strabismus eye is a left eye, the stereoscopic vision training providing apparatus 100 may adjust training coordinates of an area corresponding to the left eye. When the strabismus eye is a right eye, the stereoscopic vision training providing apparatus 100 may adjust training coordinates of an area corresponding to the right eye.

Referring to FIG. 13, a training coordinate value of an area corresponding to the strabismus eye according to stereoscopic vision may be identified. For example, when the stereoscopic vision is 1600 arcsec, the training coordinate value may be adjusted by adding or subtracting 0.4444 to or from the training coordinates (k,0) of the area corresponding to the strabismus eye. Moreover, when the stereoscopic vision is 800 arcsec, the training coordinate value may be adjusted by adding or subtracting 0.2222 to or from the training coordinates (k,0) of the area corresponding to the strabismus eye.

In the meantime, in the table of FIG. 13, the number of difficulties, information about pieces of stereoscopic vision according to difficulty, and a training coordinate correction value according to stereoscopic vision are not limited to the examples. For example, it is obvious that the number of difficulties may be smaller or greater than 10, stereoscopic vision values according to difficulty, and training coordinate correction values according to stereoscopic vision may be set to values different from values in the examples.

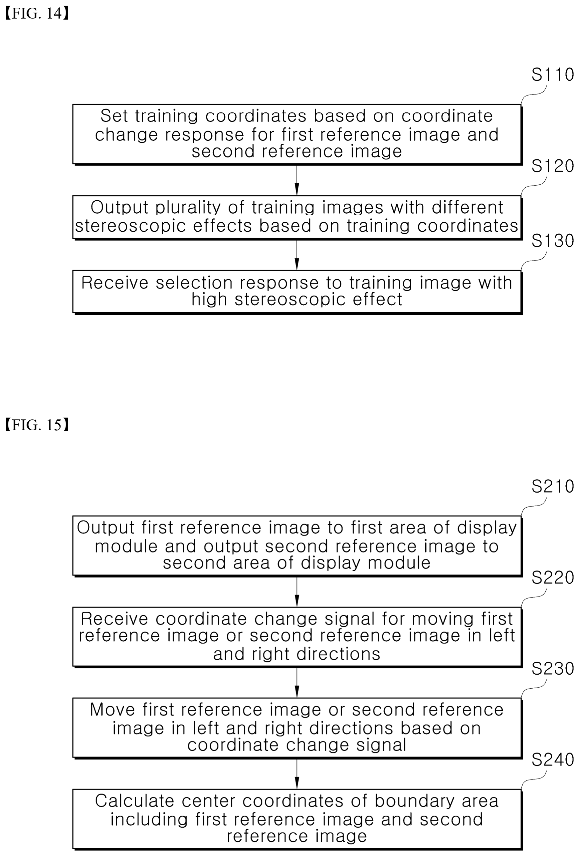

FIG. 14 is a flowchart showing a method of providing stereoscopic vision training, according to an embodiment of the present disclosure.

Referring to FIG. 14, the stereoscopic vision training providing apparatus 100 may set training coordinates based on a coordinate change response for a first reference image and a second reference image (S110). The stereoscopic vision training providing apparatus 100 may output the first reference image to an area corresponding to a left eye through the display module 150 and may output the second reference image to an area corresponding to a right eye through the display module 150. In addition, the stereoscopic vision training providing apparatus 100 may receive a coordinate change signal for completing a specific shape by moving the first reference image or the second reference image in the left and right directions through the input module 130. Besides, the stereoscopic vision training providing apparatus 100 may calculate training coordinates based on the input signal.

In addition, the stereoscopic vision training providing apparatus 100 may output a plurality of training images with different stereoscopic effects based on training coordinates (S120). In detail, the stereoscopic vision training providing apparatus 100 may output the plurality of training images with different stereoscopic effects to both the area corresponding to the left eye and the area corresponding to the right eye. The stereoscopic vision training providing apparatus 100 may output a plurality of training images by adjusting training coordinates based on stereoscopic vision information of a training image to be used in each training. The stereoscopic vision training providing apparatus 100 may output stationary training images in a static stereoscopic vision training operation and may output moving training images in a dynamic stereoscopic vision training operation.

Moreover, the stereoscopic vision training providing apparatus 100 may receive a selection response to a training image with high stereoscopic effect (S130). In detail, the stereoscopic vision training providing apparatus 100 may receive a response for selecting images in descending order of a stereoscopic effect in a static stereoscopic vision training operation and may receive a response for selecting an image with a high stereoscopic effect in a dynamic stereoscopic vision training operation.

Furthermore, the stereoscopic vision training providing apparatus 100 may adjust the difficulty of the next training based on the selection response of a trainee.

FIG. 15 is a flowchart showing a method for setting training coordinates of the present disclosure.

Referring to FIG. 15, the stereoscopic vision training providing apparatus 100 may output a first reference image to a first area and may output a second reference image to a second area (S210). In detail, the stereoscopic vision training providing apparatus 100 may output the first reference image to the first area (i.e., an area corresponding to a left eye) of the display module 150 and may output the second reference image to the second area (i.e., an area corresponding to a right eye) of the display module 150. The first area and the second area of the display module 150 may be divided based on locations within one display. According to an embodiment, the first area and the second area may mean a plurality of displays that are physically separated from each other.

Moreover, the stereoscopic vision training providing apparatus 100 may receive a coordinate change signal for moving the first reference image 210 or the second reference image 220 in left and right directions (S220). In detail, the stereoscopic vision training providing apparatus 100 may receive a coordinate change signal for moving an image corresponding to a strabismus eye in the left and right directions. For example, when a trainee's left eye is strabismus, the coordinate change signal for the first reference image 210 corresponding to the left eye may be received. Furthermore, when the trainee's right eye is strabismus, the coordinate change signal for the second reference image 220 corresponding to the right eye may be received.

Also, the stereoscopic vision training providing apparatus 100 may move the first reference image 210 or the second reference image 220 in the left and right directions based on the coordinate change signal (S230). In particular, the stereoscopic vision training providing apparatus 100 may move the location of the first reference image 210 or the second reference image 220, which are output to the display module 150, in real time depending on the received coordinate change signal. Accordingly, the trainee may determine whether the first reference image 210 and the second reference image 220 have a reference shape by movement, through the display module 150. When the reference shape is identified, the trainee may enter a signal indicating that the coordinate change has been completed. The stereoscopic vision training providing apparatus 100 may proceed to the next step when a completion signal is received.

Moreover, the stereoscopic vision training providing apparatus 100 may calculate the center coordinates of the boundary area including the first reference image 210 and the second reference image 220 (240). In detail, the stereoscopic vision training providing apparatus 100 may calculate the center coordinates of the boundary area, which includes the first reference image 210 and the second reference image 220 and which has the minimum size. Also, the stereoscopic vision training providing apparatus 100 may set the calculated center coordinates as training coordinates of an area corresponding to the strabismus eye.

In accordance with the stereoscopic vision training providing apparatus 100 of the present disclosure, it is possible to proactively perform a training coordinate setting operation through measuring the strabismus angle, thereby increasing the effect of stereoscopic vision training by accurately reflecting the degree of strabismus angle according to the trainee's status while the trainee is training.

Moreover, in accordance with the stereoscopic vision training providing apparatus 100 of the present disclosure, it is possible to provide not only static stereoscopic vision training but also dynamic stereoscopic vision training, thereby increasing the effect of a trainee's stereoscopic vision training.

Meanwhile, the disclosed embodiments may be implemented in a form of a recording medium storing instructions executable by a computer. The instructions may be stored in a form of program codes, and, when executed by a processor, generate a program module to perform operations of the disclosed embodiments. The recording medium may be implemented as a computer-readable recording medium.

The computer-readable recording medium may include all kinds of recording media in which instructions capable of being decoded by a computer are stored. For example, there may be read only memory (ROM), random access memory (RAM), magnetic tape, magnetic disk, flash memory, optical data storage device, and the like.

Disclosed embodiments are described above with reference to the accompanying drawings. One ordinary skilled in the art to which the present disclosure belongs will understand that the present disclosure may be practiced in forms other than the disclosed embodiments without altering the technical ideas or essential features of the present disclosure. The disclosed embodiments are examples and should not be construed as limited thereto.

## Claims

1. A stereoscopic vision training providing apparatus, the apparatus comprising:
a display module;
a memory including a first reference image, a second reference image, a plurality of training images, stereoscopic vision information;
an input module configured to receive a response of a trainee; and
a control module configured to:
output the first reference image to a first area of the display module, and output the second reference image to a second area of the display module;
set training coordinates based on a coordinate change signal for the first reference image and the second reference image;
output the plurality of training images with different stereoscopic effects to the display module based on the training coordinates; and
perform a stereoscopic vision training operation of receiving a selection response to a training image with a high stereoscopic effect.

2. The apparatus of claim 1, wherein the first area is an area corresponding to a left eye of the trainee,
wherein the second area is an area corresponding to a right eye of the trainee, and
wherein the coordinate change signal is a signal for moving the first reference image or the second reference image in left and right directions.

3. The apparatus of claim 1, wherein the control module is configured to:
move the first reference image or the second reference image depending on the coordinate change signal;
calculate center coordinates of a boundary area including the first reference image and the second reference image; and
set the training coordinates based on the calculated center coordinates.

4. The apparatus of claim 1, wherein the control module is configured to:
correct the training coordinates of at least one of the plurality of training images based on the stereoscopic vision information; and
correct training coordinates of the plurality of training images output to an area corresponding to a strabismus eye based on the stereoscopic vision information.

5. The apparatus of claim 1, wherein the stereoscopic vision information has different stereoscopic vision values depending on difficulty for each training of the stereoscopic vision training operation.

6. The apparatus of claim 1, wherein the stereoscopic vision training operation includes:
static stereoscopic vision training configured to select a training image in descending order of a stereoscopic effect among the plurality of training images, which do not move; and
dynamic stereoscopic vision training configured to select a training image with a high stereoscopic effect among the plurality of training images, which move.

7. The apparatus of claim 1, wherein the control module is configured to:
perform the stereoscopic vision training operation multiple times; and
adjust difficulty of a stereoscopic vision training operation in a next sequence based on a training result of a stereoscopic vision training operation in a previous sequence.

8. A stereoscopic vision training providing method performed by a control module of an apparatus, the method comprising:
outputting a first reference image to a first area of a display module, and outputting a second reference image to a second area of the display module;
setting training coordinates based on a coordinate change signal for the first reference image and the second reference image;
outputting a plurality of training images with different stereoscopic effects to the display module based on the training coordinates; and
performing a stereoscopic vision training operation of receiving a selection response to a training image with a high stereoscopic effect.

9. The method of claim 8, wherein the first area is an area corresponding to a left eye of a trainee,
wherein the second area is an area corresponding to a right eye of the trainee, and
wherein the coordinate change signal is a signal for moving the first reference image or the second reference image in left and right directions.

10. The method of claim 8, wherein the setting of the training coordinates includes:
moving the first reference image or the second reference image depending on the coordinate change signal;
calculating center coordinates of a boundary area including the first reference image and the second reference image; and
setting the training coordinates based on the calculated center coordinates.

11. The method of claim 8, further comprising:
correcting the training coordinates of at least one of the plurality of training images based on the stereoscopic vision information; and
correcting training coordinates of the plurality of training images output to an area corresponding to a strabismus eye based on the stereoscopic vision information.

12. The method of claim 8, wherein the stereoscopic vision information has different stereoscopic vision values depending on difficulty for each training of the stereoscopic vision training operation.

13. The method of claim 8, wherein the stereoscopic vision training operation includes:
static stereoscopic vision training configured to select a training image in descending order of a stereoscopic effect among the plurality of training images, which do not move; and
dynamic stereoscopic vision training configured to select a training image with a high stereoscopic effect among the plurality of training images, which move.

14. The method of claim 8, further comprising:
performing the stereoscopic vision training operation multiple times; and
adjusting difficulty of a stereoscopic vision training operation in a next sequence based on a training result of a stereoscopic vision training operation in a previous sequence.

15. A computer-readable recording medium storing a computer program for performing the stereoscopic vision training providing method of claim 8.
